# EUROPEAN PATENT APPLICATION

(11) **EP 4 741 426 A1**
(43) Date of publication of application: **13.05.2026**
(21) Application number: 24838721.9
(22) Date of filing: 05.07.2024
(51) Int. Cl.: C07K 19/00, C07K 14/54, C07K 16/28, C12N 15/85, C12N 5/10

(54) **COMPLEX OF ANTIBODY AND IL-2 AND USE THEREOF**

(30) Priority: 07.07.2023 CN 202310832340
(71) Applicant: Leto Laboratories Co., Ltd, Beijing 100094 (CN)
(72) Inventor: GAO, Xiang, Beijing 100094 (CN); WANG, Guoyong, Beijing 100094 (CN); WEI, Tingting, Beijing 100094 (CN); TAN, Wei, Beijing 100094 (CN); ZHANG, Ting, Beijing 100094 (CN); ZHU, Xiaoting, Beijing 100094 (CN); DU, Wenyu, Beijing 100094 (CN); ZHANG, Jianjun, Beijing 100094 (CN); ZHANG, Wei, Beijing 100094 (CN)
(74) Representative: Gille Hrabal Partnerschaftsgesellschaft mbB Patentanwälte
(86) International application number: PCT/CN2024/103902
(87) International publication number: WO 2025/011468

(57) **Abstract**

The present invention relates to the technical field of antibodies, and in particular, to a complex of an antibody and IL-2 and a use thereof. Provided is a complex of an antibody and IL-2, comprising: an immune checkpoint antibody and an IL15Rα receptor domain that are covalently linked, and an IL-2/15 chimera (S-unit molecule) or a variant thereof that is non-covalently compounded with the IL15Rα receptor domain. By means of design, IL-2 molecules are gradually dissociated from the complex to achieve a short half-life period, and the immune checkpoint antibody achieves a long half-life period due to the fact that an Fc domain is recycled by binding to FcRn. Therefore, the half-life period and the administration dosage of the two molecules can be matched with each other.

## Description

### CROSS-REFERENCE TO RELATED APPLICATIONS

The present application claims priority to the prior application with the Patent Application No. 202310832340X and entitled "COMPLEX OF ANTIBODY AND IL-2 AND USE THEREOF" filed with the China National Intellectual Property Administration on July 07, 2023, which is incorporated herein by reference in its entirety.

### TECHNICAL FIELD

The present disclosure relates to the technical field of antibodies, and in particular to a complex of an antibody and IL-2 and use thereof.

### BACKGROUND

Cancer is a disease that seriously compromises the human health and life expectancy, which is characterized by the overgrowth of malignant tumor cells and may cause a variety of complications. Since entering the 21st century, tumor immunotherapy represented by PD-1/PD-L1 has revolutionized tumor immunotherapy and achieved great success. The development of new tumor immunotherapy targets and combination administration regimens has become one of the biggest hotspots in new drug research and development in China and even the world. However, with the accumulation of more clinical data on a series of PD-1/PD-L1 antibodies such as Keytruda and Opdivo, the limitations of PD-1/PD-L1 antibodies in the field of solid tumor treatment have become increasingly apparent.

Cytokines, as naturally occurring immune cell agonists, exhibit highly promising potential in enhancing the overall immune system and antagonizing tumors. However, the great toxic and side effects associated with them greatly restrict the clinical use of such molecules. Therefore, while cytokines represented by modified IL-2 have become the top priority in the pipeline layout of numerous pharmaceutical companies, various design strategies for reducing toxicity and enhancing efficacy for IL-2 and IL-15 have been disclosed. In 2022, the Chinese Patent No. CN114380919A discloses a brand-new IL-2 design strategy. In this strategy, the sequence responsible for binding to CD25 (i.e., IL-2Rα) and some other sequences in the IL-2 molecule are replaced with the corresponding sequences in IL-15, forming a brand-new cytokine (S-unit molecule) that binds to the CD215 fragment (IL-15Rα) to form a stable complex (L-unit molecule). The complex L-unit is a brand-new cytokine with strong cytological activity and anti-tumor activity.

In 2021, Roche reported that a fusion protein of PD-1 antibody and IL-2 mutant (IL-2v) has stronger anti-tumor activity than the PD-1 antibody alone and can overcome immune checkpoint tolerance. In September 2022, Roche published a study entitled "PD-1-cis IL-2R agonism yields better effectors from stem-like CD8+ T cells" in Nature again. In various *in vivo* tumor efficacy models, the study confirmed that compared to PD-1 antibody monotherapy, IL-2v monotherapy, and combination therapy thereof, the PD1-IL2v fusion protein exhibits stronger tumor growth inhibition capability, has better survival benefits, and significantly expands the number of antigen-specific PD-1+ TCF-1+ stem-like T cells. Although both biological mechanisms and preclinical efficacy experiments have shown that the PD1-IL2v fusion protein has stronger anti-tumor activity than PD-1 antibody or IL-2v alone, many problems exist in the actual design of the drug molecule. In one aspect, there is a huge difference in the half-lives of the PD-1 antibody and IL-2 in humans. According to literature reports, the half-life of the PD-1 antibody in humans can be as long as 20 days (Teresa C. Longoria et al., Expert Opin Drug Metab Toxicol. 2016 Oct; 12: 1247-1253), whereas the half-life of aldesleukin (wild-type IL-2) in humans is only about 5-7 minutes (M T Lotze et al., J Immunol. 1985 Jan; 134(1): 157-66.), with a difference of 3000-fold or higher between the two, which makes them unsuitable for direct development as a fusion protein. In another aspect, there is also a huge difference in the administration dosages of PD-1 antibody and IL-2 molecule in clinical practice. Clinically, the administration dose of the PD-1 antibody (Keytruda) is 200-400 mg each time, equivalent to about 2-5 mg/kg, whereas the administration dose of wild-type IL-2 (aldesleukin) is only 0.037 mg/kg, with a difference of 50-100-fold between the two, which also makes them unsuitable for direct development as a fusion protein.

Therefore, there is a need in the art for a product that can match an immune checkpoint antibody and a cytokine in terms of half-life and administration dose to achieve uniformity.

### SUMMARY

In view of this, in a first aspect, the present disclosure provides a complex of an antibody and IL-2, comprising:
an immune checkpoint antibody and an IL15Rα receptor domain that are covalently linked, and
an IL-2/15 chimera (S-unit molecule) non-covalently complexed with the IL15Rα receptor domain, or a variant thereof.

In some specific embodiments, the IL-2/15 chimera comprises substituting a region in an IL-2 molecule that mediates binding of the IL-2 molecule to IL2Rα with a region in an IL-15 molecule that mediates binding of the IL-15 molecule to IL15Ra.

Further, the region in the IL-15 molecule that mediates binding of the IL-15 molecule to IL15Rα comprises 9 or more amino acid residues.

In some specific embodiments, the region in the IL-15 molecule that mediates binding of the IL-15 molecule to IL15Rα is a contiguous amino acid sequence.

In some specific embodiments, the region in the IL-15 molecule that mediates binding of the IL-15 molecule to IL15Rα is a non-contiguous amino acid sequence.

In some specific embodiments, the region in the IL-15 molecule that mediates binding of the IL-15 molecule to IL15Rα comprises 1 or more non-contiguous amino acid sites. In some specific embodiments, the region in the IL-2 molecule that mediates binding of the IL-2 molecule to IL2Rα is a contiguous amino acid sequence.

In some specific embodiments, the region in the IL-2 molecule that mediates binding of the IL-2 molecule to IL2Rα is a non-contiguous amino acid sequence.

In some specific embodiments, the region in the IL-2 molecule that mediates binding of the IL-2 molecule to IL2Rα comprises 1 or more non-contiguous amino acid sites.

In some specific embodiments, the IL-2/15 chimera can reduce the affinity of the IL-2 molecule for IL2Rα.

In some specific embodiments, the IL-2/15 chimera can eliminate the affinity of the IL-2 molecule for IL2Rα.

In some specific embodiments, the IL-2/15 chimera comprises one or more of:
substituting loop M or a variant thereof in the region that mediates binding of the IL-2 molecule to IL2Rα with loop m or a variant thereof in the region that mediates binding of the IL-15 molecule to IL15Rα;
substituting helix B or a variant thereof with helix b or a variant thereof in the region that mediates binding of the IL-15 molecule to IL15Rα;
substituting loop N or a variant thereof in the region that mediates binding of the IL-2 molecule to IL2Rα with loop n or a variant thereof in the region that mediates binding of the IL-15 molecule to IL15Rα;
substituting helix C or a variant thereof with helix c or a variant thereof in the region that mediates binding of the IL-15 molecule to IL15Rα; or
substituting loop X or a variant thereof in the region that mediates binding of the IL-2 molecule to IL2Rα with loop x or a variant thereof in the region that mediates binding of the IL-15 molecule to IL15Rα.

In some specific embodiments, the region that mediates binding of the IL-2 molecule to IL2Rα comprises loop M or a variant thereof, wherein loop M comprises a sequence set forth in SEQ ID NO. 1.

In some specific embodiments, the region that mediates binding of the IL-2 molecule to IL2Rα comprises loop N or a variant thereof, wherein loop N comprises a sequence set forth in SEQ ID NO. 5.

In some specific embodiments, the region that mediates binding of the IL-2 molecule to IL2Rα comprises helix B or a variant thereof, wherein helix B comprises a sequence set forth in SEQ ID NO. 2.

In some specific embodiments, the region that mediates binding of the IL-2 molecule to IL2Rα comprises loop M or a variant thereof, and helix B or a variant thereof, wherein loop M comprises a sequence set forth in SEQ ID NO. 1, and helix B comprises a sequence set forth in SEQ ID NO. 2.

In some specific embodiments, the region that mediates binding of the IL-2 molecule to IL2Rα comprises helix B or a variant thereof, and loop N or a variant thereof, wherein helix B comprises a sequence set forth in SEQ ID NO. 2, and loop N comprises a sequence set forth in SEQ ID NO. 5.

In some specific embodiments, the region that mediates binding of the IL-2 molecule to IL2Rα comprises loop M or a variant thereof, and loop N or a variant thereof, wherein loop M comprises a sequence set forth in SEQ ID NO. 1, and loop N comprises a sequence set forth in SEQ ID NO. 5.

In some specific embodiments, the region that mediates binding of the IL-2 molecule to IL2Rα sequentially comprises, from the N-terminus to the C-terminus: loop M or a variant thereof, helix B or a variant thereof, and loop N or a variant thereof, wherein loop M comprises a sequence set forth in SEQ ID NO. 1, helix B comprises a sequence set forth in SEQ ID NO. 2, and loop N comprises a sequence set forth in SEQ ID NO. 5.

In some specific embodiments, the region that mediates binding of the IL-15 molecule to IL15Rα comprises loop m or a variant thereof, wherein loop m comprises a sequence set forth in SEQ ID NO. 8.

In some specific embodiments, the region that mediates binding of the IL-15 molecule to IL15Rα comprises loop n or a variant thereof, wherein loop n comprises a sequence set forth in SEQ ID NO. 12.

In some specific embodiments, the region that mediates binding of the IL-15 molecule to IL15Rα comprises helix b or a variant thereof, wherein helix b comprises a sequence set forth in SEQ ID NO. 9.

In some specific embodiments, the region that mediates binding of the IL-15 molecule to IL15Rα comprises loop m or a variant thereof, and helix b or a variant thereof, wherein loop m comprises a sequence set forth in SEQ ID NO. 8, and helix b comprises a sequence set forth in SEQ ID NO. 9.

In some specific embodiments, the region that mediates binding of the IL-15 molecule to IL15Rα comprises helix b or a variant thereof, and loop n or a variant thereof, wherein helix b comprises a sequence set forth in SEQ ID NO. 9, and loop n comprises a sequence set forth in SEQ ID NO. 12.

In some specific embodiments, the region that mediates binding of the IL-15 molecule to IL15Rα comprises loop m or a variant thereof, and loop n or a variant thereof, wherein loop m comprises a sequence set forth in SEQ ID NO. 8, and loop n comprises a sequence set forth in SEQ ID NO. 12.

In some specific embodiments, the region that mediates binding of the IL-15 molecule to IL15Rα sequentially comprises, from the N-terminus to the C-terminus: loop m or a variant thereof, helix b or a variant thereof, and loop n or a variant thereof, wherein loop m comprises a sequence set forth in SEQ ID NO. 8, helix b comprises a sequence set forth in SEQ ID NO. 9, and loop n comprises a sequence set forth in SEQ ID NO. 12.

In some specific embodiments, the IL-2/15 chimera comprises substituting loop M or a variant thereof in the region that mediates binding of the IL-2 molecule to IL2Rα with loop m or a variant thereof in the region that mediates binding of the IL-15 molecule to IL15Rα.

In some specific embodiments, the IL-2/15 chimera comprises substituting helix B or a variant thereof in the region that mediates binding of the IL-2 molecule to IL2Rα with helix b or a variant thereof in the region that mediates binding of the IL-15 molecule to IL15Rα.

In some specific embodiments, the IL-2/15 chimera comprises substituting loop N or a variant thereof in the region that mediates binding of the IL-2 molecule to IL2Rα with loop n or a variant thereof in the region that mediates binding of the IL-15 molecule to IL15Rα.

In some specific embodiments, the IL-2/15 chimera comprises substituting loop M or a variant thereof in the region that mediates binding of the IL-2 molecule to IL2Rα with loop m or a variant thereof in the region that mediates binding of the IL-15 molecule to IL15Rα, and substituting helix B or a variant thereof in the region that mediates binding of the IL-2 molecule to IL2Rα with helix b or a variant thereof in the region that mediates binding of the IL-15 molecule to IL15Rα.

In some specific embodiments, the IL-2/15 chimera comprises substituting helix B or a variant thereof in the region that mediates binding of the IL-2 molecule to IL2Rα with helix b or a variant thereof in the region that mediates binding of the IL-15 molecule to IL15Rα, and substituting loop N or a variant thereof in the region that mediates binding of the IL-2 molecule to IL2Rα with loop n or a variant thereof in the region that mediates binding of the IL-15 molecule to IL15Rα.

In some specific embodiments, the IL-2/15 chimera comprises substituting loop M or a variant thereof in the region that mediates binding of the IL-2 molecule to IL2Rα with loop m or a variant thereof in the region that mediates binding of the IL-15 molecule to IL15Rα, and substituting loop N or a variant thereof in the region that mediates binding of the IL-2 molecule to IL2Rα with loop n or a variant thereof in the region that mediates binding of the IL-15 molecule to IL15Rα.

In some specific embodiments, the IL-2/15 chimera comprises substituting loop M or a variant thereof in the region that mediates binding of the IL-2 molecule to IL2Rα with loop m or a variant thereof in the region that mediates binding of the IL-15 molecule to IL15Rα, substituting helix B or a variant thereof with helix b or a variant thereof in the region that mediates binding of the IL-15 molecule to IL15Rα, and substituting loop N or a variant thereof in the region that mediates binding of the IL-2 molecule to IL2Rα with loop n or a variant thereof in the region that mediates binding of the IL-15 molecule to IL15Rα.

In some specific embodiments, the IL-2/15 chimera enables the modified IL-2 molecule to bind to IL15Rα or a sushi domain of IL15Rα.

In some specific embodiments, the IL-2/15 chimera further comprises substituting loop X or a variant thereof in the IL-2 molecule with loop x or a variant thereof in the IL-15 molecule, wherein loop x comprises a sequence set forth in SEQ ID NO. 10, and loop X comprises a sequence set forth in SEQ ID NO. 3.

In some specific embodiments, the IL-2/15 chimera further comprises substituting helix C or a variant thereof in the IL-2 molecule with helix c or a variant thereof in the IL-15 molecule, wherein helix c comprises a sequence set forth in SEQ ID NO. 11, and helix C comprises a sequence set forth in SEQ ID NO. 4.

In some specific embodiments, the substitution can improve the stability of the IL-2/15 chimera, compared to an IL-2/15 chimera that does not comprise the substitution.

In some specific embodiments, the IL-2/15 chimera further comprises an amino acid replacement in a helix D region of the IL-2 molecule, wherein the replaced amino acid is capable of forming an aromatic ring interaction with phenylalanine (F) at position 43 in helix b to stabilize the hydrophobic core, wherein the position of the amino acid is determined based on an amino acid sequence of a natural IL-15 molecule according to the EU index of KABAT numbering; helix D comprises a sequence set forth in SEQ ID NO. 6.

In some specific embodiments, the amino acid sequence of the natural IL-15 molecule is set forth in SEQ ID NO. 13.

In some specific embodiments, the amino acid replacement includes W121F, wherein the position of the amino acid is determined based on an amino acid sequence of a natural IL-2 molecule according to the EU index of KABAT numbering.

In some specific embodiments, the amino acid sequence of the natural IL-2 molecule is set forth in SEQ ID NO. 7.

In some specific embodiments, the IL-2/15 chimera comprises a sequence set forth in SEQ ID NO. 14.

In one specific embodiment, the IL-2/15 chimera may be a variant sequence comprising one or more amino acid substitutions of N77D, E76Q, I80T, and/or L81T; and/or
a variant having one, two, or three amino acid deletions at the C-terminus.

In some specific embodiments, the IL-2/15 chimera may be a variant sequence comprising an N77D amino acid substitution.

In one specific embodiment, the IL-2/15 chimera may be a variant sequence comprising an E76Q amino acid substitution.

In one specific embodiment, the IL-2/15 chimera may be a variant sequence comprising an I80T amino acid substitution.

In one specific embodiment, the IL-2/15 chimera may be a variant sequence comprising an L81T amino acid substitution.

In some specific embodiments, the IL-2/15 chimera comprises sequences set forth in SEQ ID NOs: 23-25.

In some specific embodiments, the IL-2/15 chimera variant has sequences set forth in SEQ ID NOs: 15-18.

By mutating the IL-2/15 chimera, the activity of the IL-2/15 chimera is reduced, so that the administration dose of the fusion protein can be further increased.

In some specific embodiments, the IL15Rα receptor domain comprises a sushi domain or a variant thereof.

In some specific embodiments, the sushi domain or the variant thereof comprises a sequence set forth in SEQ ID NO. 19.

In some specific embodiments, the IL15Rα receptor domain is linked to the C-terminus of the heavy chain of the immune checkpoint antibody.

In some specific embodiments, the immune checkpoint antibody and the IL15Rα receptor domain are covalently linked by means of fusion.

Still further, the fusion is achieved via a polypeptide linker protein.

Still further, the polypeptide linker protein comprises at least about 4 amino acids, at least about 5 amino acids, at least about 6 amino acids, at least about 7 amino acids, at least about 8 amino acids, at least about 9 amino acids, at least about 10 amino acids, at least about 11 amino acids, at least about 12 amino acids, at least about 13 amino acids, at least about 14 amino acids, at least about 15 amino acids, at least about 16 amino acids, at least about 17 amino acids, at least about 18 amino acids, at least about 19 amino acids, at least about 20 amino acids, or at least about 21 amino acids.

Still further, the polypeptide linker protein is (G₄S)ₙ, wherein n is an integer.

In some specific embodiments, n = 2.

In some specific embodiments, the immune checkpoint antibody is an antibody targeting a programmed death receptor and a ligand thereof.

In some specific embodiments, the immune checkpoint antibody is an antibody targeting PD-1, PD-L1, CTLA-4, CD4, CD40, CD80, TNF, CD86, 4-1BB/CD137, B7-H3/CD276, LMTK3, LAG3, TIM-3, IDO1, or TIGIT.

In some specific embodiments, the immune checkpoint antibody is an antibody targeting PD-1, PD-L1, or CTLA-4.

In some specific embodiments, the immune checkpoint antibody is a PD-1 antibody.

In some specific embodiments, the PD-1 antibody may be of any origin, e.g., human, murine, rabbit, camelid, etc.

In one specific embodiment, the PD-1 antibody is of murine origin.

Further, a light chain LC of the PD-1 antibody is set forth in SEQ ID NO. 20.

Further, a fusion protein of a heavy chain HC of the PD-1 and the IL15Rα receptor domain (CD215 fragment) (sushi domain) has a sequence set forth in SEQ ID NO. 21. In a second aspect, the present disclosure provides a nucleic acid molecule encoding the complex described herein.

In a third aspect, the present disclosure provides a nucleic acid molecule combination comprising: a nucleic acid molecule encoding the immune checkpoint antibody and IL15Rα receptor domain that are covalently linked described herein; and a nucleic acid molecule encoding the IL-2/15 chimera described herein.

In a fourth aspect, the present disclosure provides a vector comprising the nucleic acid molecule described herein or the combination of nucleic acid molecules described herein.

Further, the nucleic acid molecule and/or the nucleic acid molecule combination encoding the immune checkpoint antibody and IL15Rα receptor domain that are covalently linked described herein and the IL-2/15 chimera described herein may be in different vectors or in the same vector.

In some specific embodiments, the vector of the present disclosure comprises a first vector and a second vector, wherein
the first vector comprises a nucleic acid molecule encoding the immune checkpoint antibody and IL15Rα receptor domain that are covalently linked described herein; and
the second vector comprises a nucleic acid molecule encoding the IL-2/15 chimera described herein.

In some specific embodiments, the vector of the present disclosure comprises a first vector, a second vector, and a third vector, wherein
the first vector comprises a nucleic acid molecule encoding the PD-1 light chain of the present disclosure;
the second vector comprises a nucleic acid molecule encoding the fusion protein of the PD-1 heavy chain and the IL15Rα receptor domain of the present disclosure; and
the third vector comprises a nucleic acid molecule encoding the IL-2/15 chimera described herein.

In a fifth aspect, the present disclosure provides a host cell comprising the fusion protein described herein, the nucleic acid molecule or the nucleic acid molecule combination described herein, or the vector described herein.

Further, the host cell is a mammalian cell.

Still further, the host cell is selected from a Chinese hamster ovary (CHO) cell, an HEK293 cell, a BHK cell, a murine myeloma cell (NS0 and Sp2/0), a monkey kidney (COS) cell, a VERO cell, a fibrosarcoma HT-1080 cell, and a HeLa cell.

In a sixth aspect, the present disclosure provides a pharmaceutical composition comprising the complex described herein, the nucleic acid molecule or the nucleic acid molecule combination described herein, the vector described herein, or the host cell described herein, and a pharmaceutically acceptable excipient.

In a seventh aspect, the present disclosure provides use of the complex described herein, the nucleic acid molecule or the nucleic acid molecule combination described herein, the vector described herein, or the host cell described herein in the preparation of an agent for treating cancer.

Further, the cancer may be a solid tumor or a non-solid tumor, including, for example (but not limited to), gastric cancer, small intestinal cancer, sarcoma, head and neck cancer, thymic carcinoma, epithelial carcinoma, salivary gland cancer, liver cancer, cholangiocarcinoma, neuroendocrine tumor, gastric cancer, thyroid cancer, lung cancer, mesothelioma, ovarian cancer, breast cancer, prostate cancer, esophageal cancer, pancreatic cancer, glioma, renal cancer (e.g., renal cell carcinoma), bladder cancer, cervical cancer, uterine cancer, vulvar cancer, penile cancer, testicular cancer, anal cancer, choriocarcinoma, colorectal cancer, oral cancer, skin cancer, Merkel cell carcinoma, glioblastoma, brain tumor, bone cancer, eye cancer, melanoma, multiple myeloma, malignant plasmacytoma, Hodgkin's lymphoma, nodular lymphocyte-predominant Hodgkin's lymphoma, Kahler's disease, myelomatosis, plasma cell leukemia, plasmacytoma, B-cell prolymphocytic leukemia, hairy cell leukemia, B-cell non-Hodgkin's lymphoma (NHL), acute myeloid leukemia (AML), chronic lymphocytic leukemia (CLL), acute lymphoblastic leukemia (ALL), chronic myelogenous leukemia (CML), and follicular lymphoma.

In some specific embodiments, the cancer is colorectal cancer or prostate cancer.

In an eighth aspect, the present disclosure provides a method for treating cancer in a subject in need thereof, which comprises administering to the subject an effective amount of the fusion protein described herein, the nucleic acid molecule or the nucleic acid molecule combination described herein, the vector described herein, the host cell described herein, or the pharmaceutical composition described herein.

Further, the present disclosure provides a method for killing a cancer cell in a subject in need thereof, which comprises administering to the subject an effective amount of the fusion protein described herein, the nucleic acid molecule or the nucleic acid molecule combination described herein, the vector described herein, the host cell described herein, or the pharmaceutical composition described herein.

Further, the present disclosure comprises administering to the subject a second anticancer therapy, including a therapy selected from chemotherapy, radiotherapy, surgery, an agent that activates innate immune cells, an agent that enhances the survival of NK and/or CD8+ T cells, an agent that inhibits Tregs (T regulatory cells), TAMs (tumor-associated macrophages), CAFs (cancer-associated fibroblasts), or MDSCs (myeloid-derived suppressor cells), and any combination thereof.

In some specific embodiments, the cancer is colorectal cancer or prostate cancer.

### Beneficial technical effects:

By means of the design of the present disclosure, the IL-2 chimera molecule is gradually dissociated from the complex to achieve a short half-life, whereas the immune checkpoint antibody achieves a long half-life due to the Fc domain undergoing recycling by binding to FcRn. Thus, the half-lives and administration doses of the two molecules can be matched with each other.

### BRIEF DESCRIPTION OF THE DRAWINGS

FIG. 1 shows a schematic structural diagram of the fusion protein according to the present disclosure;
FIG. 2 shows the efficacy of the fusion protein and the mutant thereof according to the present disclosure in a CT-26 mouse tumor model;
FIG. 3 shows the body weight changes resulting from the fusion protein and the mutant thereof of the present disclosure in a CT-26 mouse tumor model;
FIG. 4 shows the efficacy of the fusion protein and the mutant thereof according to the present disclosure at a high dose in an RM-1 mouse tumor model; and
FIG. 5 shows the body weight changes resulting from the fusion protein and the mutant thereof according to the present disclosure in an RM-1 mouse tumor model.

### DETAILED DESCRIPTION

The present disclosure will be specifically described below with reference to specific embodiments and examples, and the advantages and various effects of the present disclosure will be more clearly presented thereby. It should be understood by those skilled in the art that these specific embodiments and examples are intended to illustrate the present disclosure rather than limit the present disclosure.

Unless otherwise stated, the starting materials and reagents used in the following examples are all commercially available products or can be prepared by using known methods. Experimental methods without specified conditions in the following examples are generally conducted under conventional conditions such as those described in Sambrook et al., Molecular Cloning: A Laboratory Manual (New York: Cold Spring Harbor Laboratory Press, 1989) or conditions recommended by the manufacturer.

Unless defined otherwise, or clear from the background, all technical and scientific terms used in the present disclosure have the same meaning as commonly understood by one of ordinary skill in the art to which the present disclosure belongs.

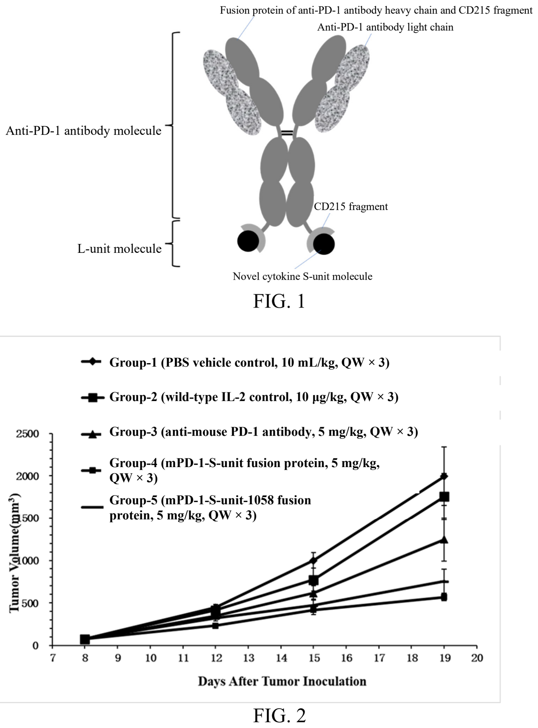

The fusion protein of the light chain of the immune checkpoint antibody and the heavy chain of the immune checkpoint antibody described herein with the IL15Rα receptor domain constitutes an immune checkpoint antibody molecule in which the IL15Rα receptor domain is fused at the C-terminus. Meanwhile, the IL-2/15 chimera (S-unit) binds to the IL15Rα receptor domain to form a non-covalent complex L-unit, as shown in FIG. 1. In the non-covalent complex L-unit molecule, the cytokine S-unit gradually dissociates from the L-unit molecule to achieve a short half-life, whereas the immune checkpoint antibody achieves a long half-life due to the Fc domain undergoing recycling by binding to FcRn.

It is known in the prior art that IL-15 interacts with IL-15 receptor α (IL-15Rα) to form a complex, and it is known in the prior art that an IL-2/15 chimera (S-unit) interacts with IL-15 receptor α (IL-15Rα) to form a complex, see CN114380919A.

In the present disclosure, the term "immune checkpoint" refers to a programmed death receptor and a ligand thereof. The immune checkpoint blockade therapy based on the programmed death receptor and the ligand thereof enhances the aggressiveness of the host immune system against tumor cells by inhibiting the binding of the programmed death receptor and the ligand thereof.

In the present disclosure, the term "antibody" refers to an immunoglobulin molecule capable of specifically binding to a target, such as a carbohydrate, a polynucleotide, a lipid, or a polypeptide, via at least one antigen recognition site located in a variable region of the immunoglobulin molecule. As used herein, unless otherwise specified, the term encompasses not only intact polyclonal or monoclonal antibodies, but also any antigen-binding portions thereof that compete for specific binding with the intact antibody, fusion proteins comprising the antigen-binding portions, and any other modified configurations of an immunoglobulin molecule comprising an antigen recognition site. Antigen-binding portions include, for example, Fab, Fab', F(ab')₂, Fd, Fv, domain antibodies (dAbs, e.g., shark and camelid antibodies), fragments comprising complementarity determining regions (CDRs), single-chain variable fragment antibodies (scFvs), maxibodies, minibodies, intrabodies, diabodies, triabodies, tetrabodies, v-NARs and bis-scFvs, and polypeptides that contain at least a portion of an immunoglobulin that is sufficient to enable specific binding of the polypeptide to an antigen. Antibodies include antibodies of any class, such as IgG, IgA, or IgM (or subclasses thereof), and the antibodies need not be of any particular class. Immunoglobulins can be classified into different classes based on the antibody amino acid sequence of the constant region of heavy chains thereof.

The "variable region" of an antibody refers to the variable region of an antibody light chain or the variable region of an antibody heavy chain, alone or in combination. As is known in the art, the variable regions of the heavy and light chains each consist of four framework regions (FRs) connected by three complementarity determining regions (CDRs), also referred to as hypervariable regions, and contribute to the formation of the antigen-binding site of antibodies. If a variant of the target variable region is desired (particularly having substitutions in amino acid residues outside the CDR regions (i.e., in the framework regions)), appropriate amino acid substitutions, preferably conservative amino acid substitutions, can be identified by comparing the target variable region with the variable regions of other antibodies that contain CDR1 and CDR2 sequences in the same canonical class as the target variable region (Chothia and Lesk, J Mol Biol 196(4): 901-917, 1987).

In certain embodiments, the definitive delineation of CDRs and the confirmation of residues comprising the binding site of an antibody are achieved by resolving the structure of the antibody and/or the structure of the antibody-ligand complex. In certain embodiments, this can be achieved by any of a variety of techniques known to those skilled in the art, such as X-ray crystallography. In certain embodiments, various analytical methods can be employed to identify or approximately identify CDR regions. In certain embodiments, various analytical methods can be employed to identify or approximately identify CDR regions. Examples of such methods include, but are not limited to, the Kabat definition, the Chothia definition, the AbM definition, the contact definition, and the conformational definition.

In the present disclosure, the term "fusion protein" refers to a first amino acid sequence linked to a second amino acid sequence, wherein the first amino acid sequence is not naturally linked to the second amino acid sequence in nature. Amino acid sequences generally present in separate proteins can be brought together in a fusion polypeptide, or amino acid sequences generally present in the same protein can be placed in a fusion polypeptide in a new arrangement. For example, a fusion protein is produced by chemical synthesis or by generating and translating a polynucleotide in which the peptide regions are encoded in the desired relationship. The fusion protein may comprise a second amino acid sequence linked to the first amino acid sequence via a peptide or polypeptide, or a peptide bond, covalent bond, non-peptide bond or non-covalent bond.

In the present disclosure, the term "C-terminus" generally refers to one of the two termini of a polypeptide chain, and the amino acid residue at this terminus carries a free α carboxyl group (-COOH).

In the present disclosure, the term "N-terminus" generally refers to one of the two termini of a polypeptide chain, and the amino acid residue at this terminus carries a free α-amino group (-NH₂).

In the present disclosure, the term "identity" refers to the number of identical matched positions shared by sequences over a comparison window, taking into account additions or deletions that must be introduced to achieve optimal alignment of the two sequences.

In the present disclosure, the term "polynucleotide" or "nucleic acid" refers to a nucleotide chain of any length and includes DNA and RNA. Nucleotides may be deoxyribonucleotides, ribonucleotides, modified nucleotides or bases and/or analogs thereof, or any substrate that can be incorporated into a strand by DNA or RNA polymerase. A polynucleotide may include modified nucleotides, such as methylated nucleotides and analogs thereof.

In the present disclosure, the term "variant" or "mutant" refers to the replacement, deletion or insertion of several amino acids in the amino acid sequence of a protein or polypeptide. Those skilled in the art know that conservative mutations of amino acids often do not affect the activity of the protein or polypeptide, and those skilled in the art also know that truncation of 1-3 amino acids at terminal groups of a protein or polypeptide generally does not affect the activity of the protein or polypeptide.

In the present disclosure, the term "effective dose" or "effective amount" refers to an amount sufficient to affect any one or more beneficial or desired results. More specifically, an effective amount prevents, alleviates or ameliorates the symptoms of a disease and/or prolongs the survival of the treated subject. For prophylactic use, the beneficial or desired results include eliminating or reducing the risk of disease, reducing the severity of disease, or delaying the onset of disease, wherein the disease includes the disease itself, complications thereof, and biochemical, histological and/or behavioral symptoms of intermediate pathological phenotypes presented during disease progression. For therapeutic use, the beneficial or desired results include clinical outcomes, such as reducing one or more symptoms of a disease.

In the present disclosure, the term "pharmaceutically acceptable carrier" or "pharmaceutically acceptable excipient" includes any material that, when combined with an active ingredient, allows the ingredient to retain biological activity and does not react with the immune system of a subject. Examples include, but are not limited to, any of the standard pharmaceutical carriers, such as phosphate buffered saline solutions, water, emulsions (such as oil/water emulsions), and various types of wetting agents. A preferred diluent for aerosol or parenteral administration is phosphate buffered saline (PBS) or normal saline (0.9%). Compositions including such carriers are formulated by well-known conventional methods (see, e.g., Remington's Pharmaceutical Sciences, 18th edition, A. Gennaro, eds., Mack Publishing Co., Easton, PA, 1990; and Remington, The Science and Practice of Pharmacy, 20th edition, Mack Publishing, 2000).

### Example 1. Gene Cloning, Construction, and Protein Expression

In the present disclosure, Suzhou Genewiz Biotechnology Co., Ltd. was entrusted to separately synthesize plasmids capable of encoding and expressing Anti-mouse PD-1 light chain LC (No. PM900, SEQ ID NO: 20), a fusion protein of Anti-mouse PD-1 heavy chain HC and a CD215 (IL-15Ra) fragment (No. PM1056, SEQ ID NO: 21), and a modified IL-2 molecule (cytokine S-unit molecule) (No. PM619, SEQ ID NO: 14), in Expi293 cells. Site-directed mutagenesis was performed according to the operation methods mentioned in "Molecular Cloning: A Laboratory Manual" to obtain plasmids capable of expressing S-unit molecule mutants (the mutation sites were N77D, E76Q, I80T, and L81T (SEQ ID NOs: 15-18), respectively; the four mutants were numbered PM1057, PM1058, PM1059, and PM1060, respectively). PM900 and PM1056 were co-expressed with plasmids PM619, PM1057, PM1058, PM1059, and PM1060, respectively, to obtain protein mPD-1-S-unit and mutants thereof. mPD-1-S-unit-1057, mPD-1-S-unit-1058, mPD-1-S-unit-1059, and mPD-1-S-unit-1060.

The inventors also found in the research that deletion mutations of 1-3 amino acids (e.g., SEQ ID NOs. 23-25) occur at the C-terminus of the S-unit molecule or mutants thereof, but such mutations do not affect activity thereof.

According to the operation methods described in "Molecular Cloning: A Laboratory Manual", DH10B was transformed and the bacterial liquid was cultured for the preparation of transient expression plasmids. Plasmids for expression were prepared according to the operation methods described in "Qiagen Mini-prep Kit" and "Qiagen Endofree Maxi-prep Kit". Expression was performed according to the standard operation procedure for transient expression. Specifically, a freshly passaged Expi293 cell suspension at 2.0-2.5 × 10⁶ cells/mL was prepared according to the volume required for transfection, and a plasmid reagent (1 µg/mL plasmid + 33.3 µL/mL Opti-MEM^{™}; for co-expression of multiple plasmids, the default is an equal mass ratio, which can be optimized) and a transfection reagent (2 µg/mL PEI + 33.3 µL/mL Opti-MEM^{™}) were prepared, and then left to stand at room temperature for 10 min. Then, the transfection reagent was added dropwise to the plasmid reagent. After 10 min of incubation at room temperature, the mixed solution was added to the cell suspension, and the resulting mixture was cultured in a shaker at 115 rpm and 36.8 °C with 5% CO₂. After 24 h, 50× KT Feed was added to a final concentration of 1×, and culturing was continued in the shaker for 5-7 days. Then, the culture was centrifuged at 8500 rpm for 15 min to collect the cell supernatant, followed by purification.

### Example 2. Purification and Analysis of mPD-1-S-Unit and Mutants Thereof

In the present disclosure, all proteins were purified by using a two-step purification method including Protein A affinity chromatography and ultrafiltration concentration with buffer exchange. The specific operation steps were as follows: 4 M sodium chloride was added to the centrifuged cell culture supernatant to a final concentration of 0.2 M sodium chloride, and the pH was adjusted to 7.5 with 1 M Tris-Cl buffer. The prepared sample was loaded onto AT Protein A Diamond (Bestchrom) pre-equilibrated with 2× PBS (pH 7.4). After loading, the column was rinsed with an equilibration buffer until the baseline stabilized to remove most of non-specifically bound impurities (such as host proteins and host DNA). Finally, the column was eluted with an elution buffer containing 100 mM glycine and 10 mM sodium chloride (pH 3.2), and the eluate was rapidly adjusted to pH 8.0. The purity of the final product was determined and analyzed by SDS-PAGE. The specific results are shown in Table 1 below.

**Table 1. Concentration and purity of purified proteins used in the present disclosure**

| Protein No. | Sequence composition | Concentration (mg/mL) | Purity |
|---|---|---|---|
| mPD-1-S-unit | Anti-mouse PD-1 light chain: PM900 | 1.8 | >90% |
| | Fusion protein of anti-mouse PD-1 heavy chain and CD215 fragment: PM1056 | | |
| | S-unit molecule: PM619 | | |
| mPD-1-S-unit-1057 | Anti-mouse PD-1 light chain: PM900 | 2.4 | >90% |
| | Fusion protein of anti-mouse PD-1 heavy chain and CD215 fragment: PM1056S-unit molecule mutant: PM1057 | | |
| mPD-1-S-unit-1058 | Anti-mouse PD-1 light chain: PM900 | 2.9 | >90% |
| | Fusion protein of anti-mouse PD-1 heavy chain and CD215 fragment: PM1056S-unit molecule mutant: PM1058 | | |
| mPD-1-S-unit-1059 | Anti-mouse PD-1 light chain: PM900 | 1.8 | >90% |
| | Fusion protein of anti-mouse PD-1 heavy chain and CD215 fragment: PM1056S-unit molecule mutant: PM1059 | | |
| mPD-1-S-unit-1060 | Anti-mouse PD-1 light chain: PM900 | 2.7 | >90% |
| | Fusion protein of anti-mouse PD-1 heavy chain and CD215 fragment: PM1056 | | |
| | S-unit molecule mutant: PM1060 | | |
| Fc-L-unit | Fc-fused CD215 fragment: SEQ ID NO: 22 | 1.5 | >90% |
| | S-unit: PM619 | | |

### Example 3: Cell Activity Assay of mPD-1-S-unit and Mutants Thereof

In the present disclosure, the HEK Blue cell line used for detecting the cell activity of mPD-1-S-unit and mutants thereof was constructed in-house by the company. The cell line can simultaneously express CD215 (IL-15Rα), CD122 (IL-15Rβ), and CD132 (IL-15Rγ) proteins. After the signaling pathway is activated, the degree of activation of the signaling pathway can be determined by detecting the absorption at 620 nm. The specific operation steps were as follows: First, the HEK Blue cell line was washed twice with PBS, and 10 mL of culture medium was added. The cells were scraped off with a cell scraper, and the cell density was adjusted to 5 × 10⁵ cells/mL after counting. Then, the cells were added to a flat-bottom 96-well culture plate at 100 µL/well, and the plate was incubated in an incubator at 37 °C with 5% CO₂ for 2 h. Then, the test samples were diluted to an initial concentration of 100 nM and then subjected to 5-fold serial dilution to obtain a total of 10 concentration points. Then, the dilutions were added to the plated cells at 100 µL/well, with 3 replicate wells set for each sample. Finally, the 96-well plate was incubated in an incubator at 37 °C with 5% CO₂ for 20 h. 20 µL of the supernatant was added to a new 96-well plate, and 180 µL of QUANTI-Blue^{™} Solution (Invivogen) was added to each well. After incubation at 37 °C for 2 h, the expression level of SEAP was detected at 620 nm using a microplate reader. The curves were fitted, and the EC50 values were calculated. The specific results are shown in Table 2.

**Table 2. Cell activity assay of mPD-1-S-unit and mutants thereof**

| Protein No. | Sequence composition | EC50 (nM) |
|---|---|---|
| mPD-1-S-unit | Anti-mouse PD-1 light chain: PM900 | 0.006 |
| | Fusion protein of anti-mouse PD-1 heavy chain and CD215 fragment: PM1056 | |
| | S-unit molecule: PM619 (S-unit) | |
| mPD-1-S-unit-1057 | Anti-mouse PD-1 light chain: PM900 | 0.33 |
| | Fusion protein of anti-mouse PD-1 heavy chain and CD215 fragment: PM1056 | |
| | S-unit molecule: PM1057 (N77D) | |
| mPD-1-S-unit-1058 | Anti-mouse PD-1 light chain: PM900 | 0.034 |
| | Fusion protein of anti-mouse PD-1 heavy chain and CD215 fragment: PM1056 | |
| | S-unit molecule: PM1058 (E76Q) | |
| mPD-1-S-unit-1059 | Anti-mouse PD-1 light chain: PM900 | 0.054 |
| | Fusion protein of anti-mouse PD-1 heavy chain and CD215 fragment: PM1056 | |
| | S-unit molecule: PM1059 (180T) | |
| mPD-1-S-unit-1060 | Anti-mouse PD-1 light chain: PM900 | 0.062 |
| | Fusion protein of anti-mouse PD-1 heavy chain and CD215 fragment: PM1056 | |
| | S-unit molecule: PM1060 (L81T) | |
| Wild-type IL-2 | SEQ ID NO:7 | 0.007 |
| Fc-L-unit molecule | SEQ ID NO: 14 and SEQ ID NO: 22 | 0.006 |

From the above cell activity assay of mPD-1-S-unit and mutants thereof based on the HEK Blue cell line, it could be found that by using different mutation strategies, the cell activity of the mPD-1-S-unit mutants was 6-50 times lower than that of the wild-type mPD-1-S-unit. The reduction in the relative activity of the cytokine is beneficial to further reducing the toxicity of the mPD-1-S-unit fusion protein and increasing the administration dose thereof, thereby achieving better efficacy.

### Example 4. Determination of Half-Life of Intact Fc-L-unit Molecule

In the present disclosure, the Fc-L-unit is composed of two portions, i.e., the S-unit and the CD215 fragment fused with Fc, and can function normally only when the two form an intact complex. Therefore, in the ELISA experiment of this example, one antibody is used to capture the S-unit, and the other antibody is used to detect the Fc portion, thereby achieving the purpose of detecting the intact Fc-L-unit molecule. In the half-life test of the intact Fc-L-unit molecule of the present disclosure, a total of 24 BALB/c mice (purchased from Beijing Vital River Laboratory Animal Technology Co., Ltd.), half male and half female, were used. The Fc-L-unit was administered via a single intravenous injection at a dose of 1.5 mg/kg and an administration volume of 5 mL/kg. Serum samples were collected from all animals before administration (D-1), immediately after the end of administration (±1 min), and 20 min, 40 min, 1 h, 2 h, 3 h, 4 h, 8 h, 24 h, and 48 h after the start of administration. Four animals/sex/group were assigned to one cohort, with cohorts used alternately. Four animals/sex were arranged for each blood collection time point. After collection, the blood samples were transferred to centrifuge tubes (without anticoagulant) and then centrifuged at 3000×g for 10 min at 4 °C. The serum was stored at below -60 °C.

The content of Fc-L-unit molecules in the serum of mice was determined by the developed ELISA method. The pharmacokinetic parameters of the administration group were calculated using the non-compartmental analysis (NCA) in WinNonlin 8.0 software. The results are shown in Table 3.

**Table 3. Calculation results of pharmacokinetic parameters**

| | t_{1/2} (h) | Tₘₐₓ (h) | Cₘₐₓ (ng/mL) | Vz | CL | MRTₗₐₛₜ |
|---|---|---|---|---|---|---|
| Mean | 1.96 | 0.03 | 31692.89 | 79.35 | 28.06 | 2.13 |
| SD | 0.02 | 0.00 | 721.69 | 7.82 | 2.48 | 0.02 |
| CV (%) | 1.02 | 0.00 | 2.28 | 9.86 | 8.84 | 0.98 |

From this experiment, it could be seen that the intact Fc-L-unit molecule is metabolized rapidly in mice. The S-unit is rapidly eliminated after dissociation with CD215, with the intact molecule having a half-life of less than two hours. The short half-life can avoid excessively strong immune activation toxicity and prevent the problem of unduly low administration dose after fusion with PD-1 antibody. Meanwhile, the molecular structure has the advantage that the half-life of the active ingredient S-unit is not affected by other structures in the molecule and does not change due to factors such as fusion with PD-1 antibody, which is beneficial to solving the problem of the difference in the half-life between the PD-1 antibody and the cytokine in the mPD-1-S-unit molecule.

### Example 5. Determination of Efficacy of mPD-1-S-unit and Mutants Thereof in CT26 Mouse Colon Cancer Tumor Model

In this example, CT26 tumor cells derived from mouse colon cancer were inoculated subcutaneously into the right dorsal region of BALB/c female mice (Beijing Vital River Laboratory Animal Technology Co., Ltd.) with an inoculation amount of 5 × 10⁵ cells/mouse. When the tumors grew to about 77 mm³, the mice were divided into 5 groups for administration, including a negative control group, an IL-2 treatment group, an anti-PD-1 treatment group, an mPD-1-S-unit treatment group, and an mPD-1-S-unit-1058 treatment group, with six animals in each group. The IL-2 treatment group was administered at a dose of 10 µg/kg, and the other groups were administered at a dose of 5 mg/kg. The administration was performed via intraperitoneal injection once a week for two weeks. The body weight of animals and tumor volume were monitored. The tumor volume was measured twice a week using a vernier caliper, and the body weight of mice was measured with an electronic balance. The long and short diameters of the tumors were measured, and the tumor volume (TV) was calculated according to the formula: TV (mm³) = 0.5 × long diameter (mm) × short diameter (mm)². The tumor growth inhibition rate (TGITV%) was calculated according to the formula: TGITV% = (1 - T/C) × 100%, where T/C = RTV of the treatment group / RTV of the control group.

At the end of the experiment, the animals were euthanized and tumor growth inhibition rates were calculated and statistically analyzed. The specific data are shown in Table 4.

**Table 4. Tumor growth inhibition rate and statistical difference analysis on day 19**

| Name of drug | Tumor growth inhibition rate on day 19 (%) | Statistical difference analysis *p* value (compared with the negative control group) |
|---|---|---|
| Wild-type IL-2 | 12.94 | 0.481 |
| Anti mouse-PD-1 | 39.66 | 0.035 |
| mPD-1-S-unit | 71.52 | 0.000 |
| mPD-1-S-unit-1058 | 62.99 | 0.001 |

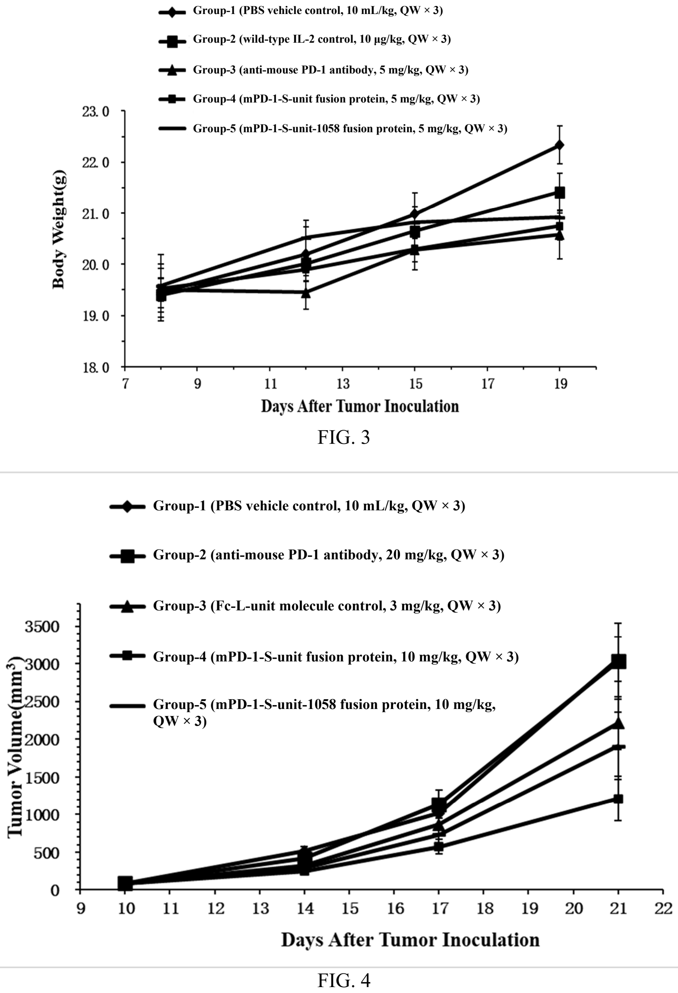

In this animal efficacy experiment, mPD-1-S-unit and mPD-1-S-unit-1058 both showed good activity against mouse colon tumor cells, with tumor volumes continuously decreasing relative to the negative control group (FIG. 2). In this experiment, the animals showed normal body weight gain, and no drug-related toxic and side effects were observed. The molecules mPD-1-S-unit and mPD-1-S-unit-1058 had similar effects on the body weight of the mice to the anti-mouse PD-1 antibody alone, and showed no additional toxicity (FIG. 3).

### Example 6. Determination of Efficacy of mPD-1-S-unit and Mutants Thereof in RM-1 Mouse Prostate Cancer Tumor Model

In this example, RM-1 tumor cells derived from mouse prostate cancer were inoculated subcutaneously into the right dorsal region of C57BL/6J female mice (Beijing Vital River Laboratory Animal Technology Co., Ltd.) with an inoculation amount of 1 × 10⁶ cells/mouse. When the tumors grew to about 87 mm³, the mice were divided into 5 groups for administration, including a negative control group, an anti-PD-1 antibody treatment group, an Fc-L-unit molecule treatment group, an mPD-1-S-unit treatment group, and an mPD-1-S-unit-1058 treatment group, with six animals in each group. The anti-PD-1 treatment group was administered at a dose of 20 mg/kg; the Fc-L-unit molecule treatment group was administered at a dose of 3 mg/kg; and the mPD-1-S-unit treatment group and the mPD-1-S-unit-1058 treatment group were administered at a dose of 10 mg/kg. The administration was performed via intraperitoneal injection once a week for two weeks. The body weight of animals and tumor volume were monitored.

The tumor volume was measured twice a week using a vernier caliper, and the body weight of mice was measured with an electronic balance. The long and short diameters of the tumors were measured, and the tumor volume (TV) was calculated according to the formula: TV (mm³) = 0.5 × long diameter (mm) × short diameter (mm)². The tumor growth inhibition rate (TGITV%) was calculated according to the formula: TGITV% = (1 - T/C) × 100%, where T/C = RTV of the treatment group / RTV of the control group.

At the end of the experiment, the animals were euthanized and tumor growth inhibition rates were calculated. The specific data are shown in Table 5.

**Table 5. Tumor growth inhibition rate on day 21**

| Name of drug | Tumor growth inhibition rate on day 21 (%) |
|---|---|
| Anti-PD-1 antibody | 4.21 |
| Fc-L-unit molecule | 28.26 |
| mPD-1-S-unit | 62.78 |
| mPD-1-S-unit-1058 | 39.73 |

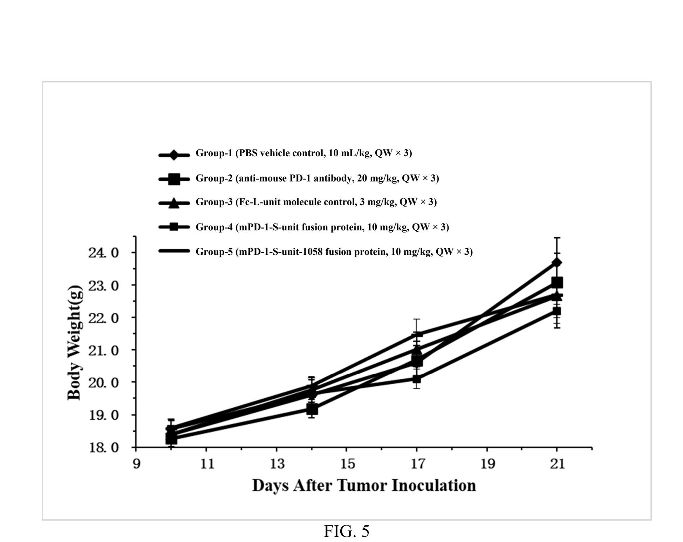

In this animal experiment, an RM-1 mouse model was used, and the efficacy and toxicity of mPD-1-S-unit and mPD-1-S-unit-1058 in this animal model were tested at a higher administration dose in the present disclosure. In this experiment, mPD-1-S-unit and mPD-1-S-unit-1058 both showed superior efficacy to the PD-1 antibody or Fc-L-unit alone (FIG. 4), and the changes in mouse body weight were similar to those of the PD-1 antibody and Fc-L-unit molecules (FIG. 5). In addition, in this experiment, except for one mouse death in the vehicle control group, no mouse death was observed in the other group, indicating that even at a high dose, mPD-1-S-unit and mPD-1-S-unit-1058 did not induce additional toxicity relative to the PD-1 antibody.

### Sequence information:

| **Sequence name** | **Sequence number** | **Sequence information** |
|---|---|---|
| IL-2 loop M amino acid sequence | SEQ ID NO:1 | NNYKNPKLTRMLTFKFYMPKKATE |
| IL-2 helix B amino acid sequence | SEQ ID NO:2 | LKHLQCLEEELKPLEEVLNL |
| IL-2 loop X amino acid sequence | SEQ ID NO:3 | AQSKNFHLR |
| IL-2 helix C amino acid sequence | SEQ ID NO:4 | PRDLISNINVIVLELK |
| IL-2 loop N amino acid sequence | SEQ ID NO:5 | GSETTFMCEYADETAT |
| IL-2 helix D amino acid sequence | SEQ ID NO:6 | IVEFLNRWITFCQSIISTLT |
| IL-2 amino acid sequence | SEQ ID NO:7 | |
| IL-15 loop m amino acid sequence | SEQ ID NO:8 | QSMHIDATLYTESDVHPSC |
| IL-15 helix b amino acid sequence | SEQ ID NO:9 | KVTAMKCFLLELQVISLE |
| IL-15 loop x amino acid sequence | SEQ ID NO:10 | SGDAS |
| IL-15 helix c amino acid sequence | SEQ ID NO:11 | IHDTVENLIILANNSL |
| IL-15 loop n amino acid sequence | SEQ ID NO:12 | SSNGNVTESGCKECEELEEKN |
| IL-15 amino acid sequence | SEQ ID NO:13 | NWVNVISDLKKIEDLIQSMHIDATLYTE SDVHPSCKVTAMKCFLLELQVISLESG DASIHDTVENLIILANNSLSSNGNVTES GCKECEELEEKNIKEFLQSFVHIVQMFI NTS |
| S-unit(PM619) | SEQ ID NO:14 | APTSSSTKKTQLQLEHLLLDLQMILNGI QSMHIDATLYTESDVHPSCKVTAMKCF LLELQVISLESGDASIHDTVENLIILANN SLSSNGNVTESGCKECEELEEKNIVEFL NRFITFAQSIISTLT |
| S-unit PM1057 N77D | SEQ ID NO:15 | APTSSSTKKTQLQLEHLLLDLQMILNGI QSMHIDATLYTESDVHPSCKVTAMKCF LLELQVISLESGDASIHDTVEDLIILANN SLSSNGNVTESGCKECEELEEKNIVEFL NRFITFAQSIISTLT |
| S-unit PM1058 E76Q) | SEQ ID NO:16 | APTSSSTKKTQLQLEHLLLDLQMILNGI QSMHIDATLYTESDVHPSCKVTAMKCF LLELQVISLESGDASIHDTVQNLIILANN SLSSNGNVTESGCKECEELEEKNIVEFL NRFITFAQSIISTLT |
| S-unit PM1059 180T | SEQ ID NO:17 | APTSSSTKKTQLQLEHLLLDLQMILNGI QSMHIDATLYTESDVHPSCKVTAMKCF LLELQVISLESGDASIHDTVENLITLAN NSLSSNGNVTESGCKECEELEEKNIVEF LNRFITFAQSIISTLT |
| S-unit PM1060 L81T | SEQ ID NO:18 | APTSSSTKKTQLQLEHLLLDLQMILNGI QSMHIDATLYTESDVHPSCKVTAMKCF LLELQVISLESGDASIHDTVENLIITANN SLSSNGNVTESGCKECEELEEKNIVEFL NRFITFAQSIISTLT |
| sushi domain | SEQ ID NO:19 | ITCPPPMSVEHADIWVKSYSLYSRERYI CNSGFKRKAGTSSLTECVLNKATNVAH WTTPSLKCIR |
| Anti-mouse PD-1 light chain LC | SEQ ID NO:20 | EIVLTQSPATLSLSPGERATLSCRASQS VSSYLAWYQQKPGQAPRLLIYDASKR ATGIPARFSGSGSGTDFTLTISSLEPEDF AVYYCDQRNNWPLTFGGGTKVEIKRT VAAPSVFIFPPSDEQLKSGTASVVCLLN NFYPREAKVQWKVDNALQSGNSQESV TEQDSKDSTYSLSSTLTLSKADYEKHK VYACEVTHQGLSSPVTKSFNRGEC |
| Fusion protein of anti-mouse PD-1 heavy chain HC and CD215 fragment | SEQ ID NO:21 | QVQLVQSGAEVKKPGASVKVSCKASG YTFTAQYMHWVRQAPGQGLEWMGIIN PSGGETGYAQKFQGRVTMTRDTSTSTV YMELSSLRSEDTAVYYCAKEGVADGY GLVDVWGQGTMVTVSSASTKGPSVFP LAPCSRSTSESTAALGCLVKDYFPEPVT VSWNSGALTSGVHTFPAVLQSSGLYSL SSVVTVPSSSLGTKTYTCNVDHKPSNT KVDKRVESKYGPPCPPCPAPEFLGGPS VFLFPPKPKDTLMISRTPEVTCVVVDVS QEDPEVQFNWYVDGVEVHNAKTKPRE EQFNSTYRVVSVLTVLHQDWLNGKEY KCKVSNKGLPSSIEKTISKAKGQPREPQ VYTLPPSQEEMTKNQVSLTCLVKGFYP SDIAVEWESNGQPENNYKTTPPVLDSD GSFFLYSRLTVDKSRWQEGNVFSCSVM HEALHNHYTQKSLSLSLGKGGGGSGG GGSITCPPPMSVEHADIWVKSYSLYSRE RYICNSGFKRKAGTSSLTECVLNKATN VAHWTTPSLKCIR |
| CD215 fragment in L-unit (fused with hIgG4Fc) | SEQ ID NO:22 | ITCPPPMSVEHADIWVKSYSLYSRERYI CNSGFKRKAGTSSLTECVLNKATNVAH WTTPSLKCIRESKYGPPCPPCPAPEFLG GPSVFLFPPKPKDTLMISRTPEVTCVVV DVSQEDPEVQFNWYVDGVEVHNAKT KPREEQFNSTYRVVSVLTVLHQDWLN GKEYKCKVSNKGLPSSIEKTISKAKGQP REPQVYTLPPSQEEMTKNQVSLTCLVK GFYPSDIAVEWESNGQPENNYKTTPPV LDSDGSFFLYSRLTVDKSRWQEGNVFS CSVMHEALHNHYTQKSLSLSLG |
| S-unit-1 | SEQ ID NO:23 | APTSSSTKKTQLQLEHLLLDLQMILNGI QSMHIDATLYTESDVHPSCKVTAMKCF LLELQVISLESGDASIHDTVENLIILANN SLSSNGNVTESGCKECEELEEKNIVEFL NRFITFAQSIISTL |
| S-unit-2 | SEQ ID NO:24 | APTSSSTKKTQLQLEHLLLDLQMILNGI QSMHIDATLYTESDVHPSCKVTAMKCF LLELQVISLESGDASIHDTVENLIILANN SLSSNGNVTESGCKECEELEEKNIVEFL NRFITFAQSIIST |
| S-unit-3 | SEQ ID NO:25 | APTSSSTKKTQLQLEHLLLDLQMILNGI QSMHIDATLYTESDVHPSCKVTAMKCF LLELQVISLESGDASIHDTVENLIILANN SLSSNGNVTESGCKECEELEEKNIVEFL NRFITFAQSIIS |

## Claims

1. A complex of an antibody and IL-2, comprising:
an immune checkpoint antibody and an IL15Rα receptor domain that are covalently linked, and
an IL-2/15 chimera or a variant thereof that is non-covalently complexed with the IL15Rα receptor domain.

2. The complex according to claim 1, wherein the IL-2/15 chimera or the variant thereof comprises one or more of:
substituting loop M (SEQ ID NO: 1) or a variant thereof in a region that mediates binding of an IL-2 molecule to IL2Rα with loop m (SEQ ID NO: 8) or a variant thereof in a region that mediates binding of an IL-15 molecule to IL15Rα;
substituting helix B (SEQ ID NO: 2) or a variant thereof with helix b (SEQ ID NO: 9) or a variant thereof in a region that mediates binding of an IL-15 molecule to IL15Rα;
substituting loop X (SEQ ID NO: 3) or a variant thereof in a region that mediates binding of an IL-2 molecule to IL2Rα with loop x (SEQ ID NO: 10) or a variant thereof in a region that mediates binding of an IL-15 molecule to IL15Rα;
substituting helix C (SEQ ID NO: 4) or a variant thereof with helix c (SEQ ID NO: 11) or a variant thereof in a region that mediates binding of an IL-15 molecule to IL15Rα; or
substituting loop N (SEQ ID NO: 5) or a variant thereof in a region that mediates binding of an IL-2 molecule to IL2Rα with loop n (SEQ ID NO: 12) or a variant thereof in a region that mediates binding of an IL-15 molecule to IL15Rα; and
optionally, performing an amino acid replacement in a helix D region of the IL-2 molecule, wherein the replaced amino acid is capable of forming an aromatic ring interaction with phenylalanine (F) at position 43 in helix b to stabilize the hydrophobic core, wherein the position of the amino acid is determined based on an amino acid sequence of a natural IL-15 molecule according to the EU index of KABAT numbering; helix D comprises a sequence set forth in SEQ ID NO. 6; and
the IL-2 wild-type molecule has a sequence set forth in SEQ ID NO: 7.

3. The complex according to claim 2, wherein the IL-2/15 chimera or the variant thereof comprises a sequence set forth in SEQ ID NO. 14.

4. The complex according to claim 2, wherein the IL-2/15 chimera or the variant thereof is a variant sequence comprising one or more amino acid substitutions of N77D, E76Q, I80T, and/or L81T;
and/or
a variant having one, two, or three amino acid deletions at the C-terminus.

5. The complex according to claim 4, wherein the IL-2/15 chimera or the variant thereof has a sequence set forth in SEQ ID NOs. 15-18 or 23-25.

6. The complex according to claim 1, wherein the IL15Rα receptor domain comprises a sushi domain or a variant thereof.

7. The complex according to claim 6, wherein the sushi domain or the variant thereof comprises a sequence set forth in SEQ ID NO. 19.

8. The complex according to claim 1, wherein the IL15Rα receptor domain is linked to the C-terminus of the heavy chain of the immune checkpoint antibody.

9. The complex according to claim 8, wherein the immune checkpoint antibody and the IL15Rα receptor domain are covalently linked by means of fusion.

10. The complex according to claim 9, wherein the fusion is achieved via a polypeptide linker protein.

11. The complex according to claim 10, wherein the polypeptide linking protein is (G₄S)ₙ, wherein n is an integer.

12. The complex according to any one of claims 1 to 11, wherein the immune checkpoint antibody is an antibody targeting a programmed death receptor and a ligand thereof.

13. The complex according to claim 12, wherein the immune checkpoint antibody is a PD-1 antibody.

14. The complex according to claim 13, wherein the PD-1 antibody light chain LC is set forth in SEQ ID NO: 20.

15. The complex according to claim 13, wherein the fusion protein of the PD-1 heavy chain HC and the IL15Rα receptor domain has a sequence set forth in SEQ ID NO: 21.

16. A nucleic acid molecule, encoding the complex according to any one of claims 1-15.

17. A nucleic acid molecule combination, comprising a nucleic acid molecule encoding the immune checkpoint antibody and the IL15Rα receptor domain that are covalently linked according to any one of claims 1-15; and a nucleic acid molecule encoding the IL-2/15 chimera according to any one of claims 1-15.

18. A vector, comprising the nucleic acid molecule according to claim 16 or the nucleic acid molecule combination according to claim 17.

19. A host cell, comprising the complex according to any one of claims 1-15, the nucleic acid molecule according to claim 16, the nucleic acid molecule combination according to claim 17, or the vector according to claim 18.

20. A pharmaceutical composition, comprising:
the complex according to any one of claims 1-15, the nucleic acid molecule according to claim 16, the nucleic acid molecule combination according to claim 17, the vector according to claim 18, or the host cell according to claim 19, and
a pharmaceutically acceptable excipient.

21. Use of the complex according to any one of claims 1-15, the nucleic acid molecule according to claim 16, the nucleic acid molecule combination according to claim 17, the vector according to claim 18, or the host cell according to claim 19 in the preparation of an agent for treating cancer.

22. The use according to claim 21, wherein the cancer is colorectal cancer or prostate cancer.
